# EUROPEAN PATENT SPECIFICATION

(11) **EP 2 260 833 B1**
(45) Date of publication and mention of the grant of the patent: **21.11.2012**
(21) Application number: 10179142.4
(22) Date of filing: 16.01.2002
(51) Int. Cl.: A61K 9/16, A61K 9/20, A61K 31/635, A61K 31/54, A61K 31/495, A61K 31/415

(54) **Bilayer pharmaceutical tablet comprising telmisartan and a diuretic**
Zweischichtiges pharmazeutisches Tablett enthaltend Telmisartan und ein Diureticum
Comprimé pharmaceutique bicouche comprenant du telmisartane et un diurétique

(43) Date of publication of application: 15.12.2010
(62) Divisional of application: 07115060.1
(73) Proprietor: Boehringer Ingelheim Pharma GmbH & Co. KG, 55216 Ingelheim am Rhein (DE)
(72) Inventor: Friedl, Thomas Dr., 88416, Ochsenhausen (DE); Schepky, Gottfried Dr., 79312, Emmendingen (DE)
(74) Representative: Simon, Elke Anna Maria

(56) References cited:
- WO-A-00/27397
- WO-A-00/43370
- LACOURCIERE Y ET AL: "COMPARISON OF THE ANTIHYPERTENSIVE EFFECTS OF A FIXED DOSE COMBINATION OF TELMISARTAN AND HYDROCHLOROTHIAZIDE VERSUS TELMISARTAN MONOTHERAPY IN MILD TO MODERATE HYPERTENSIVE PATIENTS", CANADIAN JOURNAL OF CARDIOLOGY, PULSUS GROUP, INC, XX, vol. 16, no. SUPPL F, September 2000 (2000-09), page 107F, XP001053460, ISSN: 0828-282X

## Description

### Field of invention

The present invention relates to a bilayer pharmaceutical tablet formulation for use in a method for the treatment of hypertension comprising the angiotensin II receptor antagonist telmisartan in combination with a thiazide diuretic such as hydrochlorothiazide (HCTZ).

### Background of the invention

INN Telmisartan is an angiotensin II receptor antagonist developed for the treatment of hypertension and other medical indications as disclosed in EP-A-502314.

Its chemical name is 4'-[2-n-propyl-4-methyl-6-(1-methylbenzimidazol-2-yl)-benzimidazol-1-ylmethyl]-biphenyl-2-carboxylic acid having the following structure:

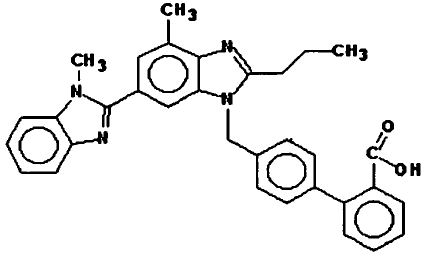

Telmisartan is generally manufactured and supplied in the free acid form. It is characterized by its very poor solubility in aqueous systems at the physiological pH range of the gastro-intestinal tract of between pH 1 to 7. As disclosed in WO 00/43370, crystalline telmisartan exists in two polymorphic forms having different melting points. Under the influence of heat and humidity, the lower melting polymorph B transforms irreversibly into the higher melting polymorph A.

Lacourciere Y. et al. Canadian Journal of Cardiology. Vol. 16. Suppl F, September 2009, page 107F discloses a telmisartan hydrochlorothiazide composition for the treament of hypertension.

Hydrochlorothiazide (HCTZ) is a thiazide diuretic which is orally administered in the treatment of edema and hypertension.

The chemical name of HCTZ is 6-chloro-3,4-dihydro-2H-1,2,4-benzothiadiazine-7-sulfonamide-1,1-dioxide having the following structure

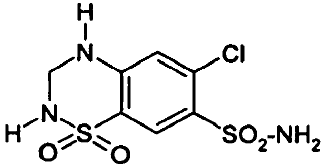

### Objects of the invention

Combination therapy of telmisartan with a diuretic like HCTZ is expected to show synergistic therapeutic efficacy in the treatment of hypertension.

It was therefore an object of the present invention to provide a fixed dose combination drug comprising telmisartan and a diuretic such as HCTZ, said combination drug displaying the required fast dissolution and immediate drug release profile combined with adequate stability.

Generally, a fixed-dose combination of drugs intended for immediate release is prepared by either making a powder mixture or a co-granulate of the two active ingredients with the necessary excipients, normally keeping the basic formulation of the corresponding mono-drug preparation and simply adding the second drug component.

With a combination of telmisartan and HCTZ, this approach was not feasible due to the incompatibility of HCTZ with basic compounds such as, e.g., meglumine (N-methyl-D-glucamine) which is a component of conventional telmisartan formulations, and the reduced dissolution rate of HCTZ from a dissolving matrix as compared with dissolution from a disintegrating tablet.

Several galenical approaches to overcome the incompatibility problem have been investigated. A classical approach is to coat the HCTZ particles in a fluidized-bed granulator with a polymer solution containing water soluble polymers like hydroxypropylcellulose, hydroxypropylmethylcellulose or polyvinylpyrrolidone, thereby reducing the contact surface area of the HCTZ particles with the telmisartan formulation during mixing and compressing. Yet, by these means it was not possible to reduce the contact area of HCTZ with the telmisartan formulation in a compressed tablet to a degree sufficient to achieve the desired prolonged shelf life.

Furthermore, the dissolution rate of HCTZ from tablets comprising coated HCTZ in a telmisartan formulation was further reduced due to the gel-forming properties of the polymer.

Another approach was to produce separate film-coated tablets for telmisartan and HCTZ in such a size and shape that these could be filled into a capsule. By dividing the doses into two to four single small tablets for telmisartan and into one or two small tablets for HCTZ, a capsule of size 1 to 0 long could be filled. Yet, with this approach the drug dissolution rate of telmisartan was reduced compared to the single entities due to a lag-time effect of the large capsule shells. Furthermore, with regard to patients' compliance a zero long capsule is not deemed reliable.

### Summary of the invention

The above-described problems associated with conventional approaches in the preparation of a fixed dose combination drug comprising telmisartan and a diuretic could be overcome by means of a bilayer pharmaceutical tablet comprising a first layer containing telmisartan in substantially amorphous form in a dissolving tablet matrix, and a second layer containing a diuretic in a disintregrating tablet matrix.

The bilayer tablet according to the present invention provides a largely pH-independent dissolution of the poorly water-soluble telmisartan, thereby facilitating dissolution of the drug at a physiological pH level, and also provides for immediate release of the diuretic from the fast disintegrating matrix. At the same time, the bilayer tablet structure overcomes the stability problem caused by the incompatibility of diuretics like HCTZ with basic constitutents of the telmisartan formulation. Producing a bilayer pharmaceutical tablet according to the invention comprises the steps of:
(i) providing a first tablet layer composition by
   a) preparing an aqueous solution of telmisartan, at least one basic agent and, optionally, a solubilizer and/or a crystallization retarder;
   b) spray-drying said aqueous solution to obtain a spray-dried granulate;
   c) mixing said spray-dried granulate with a water-soluble diluent to obtain a premix;
   d) mixing said premix with a lubricant to obtain a final blend for the first tablet layer;
   e) optionally, adding other excipients and/or adjuvants in any of steps a) to d);
(ii) providing a second tablet layer composition by
   f) mixing and/or granulating a diuretic with the constituents of a disintegrating tablet matrix and, optionally, further excipients and/or adjuvants;
   g) admixing a lubricant to obtain a final blend for the second tablet layer;
(iii) introducing the first or the second tablet layer composition in a tablet press;
(iv) compressing said tablet layer composition to form a tablet layer;
(v) introducing the other tablet layer composition into the tablet press; and
(vi) compressing both tablet layer compositions to form a bilayer tablet.

### Definitions

As used herein, the term "substantially amorphous" refers to a product comprising amorphous constituents in a proportion of at least 90%, preferably at least 95%, as determined by X-ray powder diffraction measurement.

The term "dissolving tablet matrix" refers to a pharmaceutical tablet base formulation having immediate release (fast dissolution) characteristics that readily dissolves in a physiological aqueous medium.

The term "diuretic" refers to thiazide and thiazide-analogue diuretics like hydrochlorothiazide (HCTZ), clopamide, xipamide or chlorotalidone, and any other diuretic suitable in the treatment of hypertension like, e.g., furosemide and piretanide, and combinations thereof with amiloride and triamteren.

The term "disintegrating tablet matrix" refers to a pharmaceutical tablet base formulation having immediate release characteristics that readily swells and disintegrates in a physiological aqueous medium.

### Description of the preferred embodiments

The bilayer tablet according to the present invention comprises a first layer containing telmisartan in substantially amorphous form in a dissolving tablet matrix, and a second layer containing a diuretic in a disintregrating tablet matrix.

The active ingredient telmisartan is generally supplied in its free acid form, although pharmaceutically acceptable salts may also be used. Since during subsequent processing telmisartan is normally dissolved and transformed into a substantially amorphous form, its initial crystal morphology and particle size are of little importance for the physical and biopharmaceutical properties of the bilayer tablet formulation obtained. It is however preferred to remove agglomerates from the starting material, e.g. by sieving, in order to facilitate wetting and dissolution during further processing.

Substantially amorphous telmisartan may be produced by any suitable method known to those skilled in the art, for instance, by freeze drying of aqueous solutions, coating of carrier particles in a fluidized bed, and solvent deposition on sugar pellets or other carriers. Preferably, however, the substantially amorphous telmisartan is prepared by the specific spray-drying method described hereinafter.

The other active ingredient, i.e. the diuretic, is usually employed as a fine-crystalline powder, optionally in fine-milled, peg-milled or micronized form. For instance, the particle size distribution of hydrochlorothiazide, as determined by the method of laser light scattering in a dry dispersion system (Sympatec Helos/Rodos, focal length 100 mm) is preferably as follows:
d₁₀ : ≤ 20 µm, preferably 2 to 10 µm
d₅₀ : 5 to 50 µm, preferably 10 to 30 µm
d₉₀ : 20 to 100 µm, preferably 40 to 80 µm

The bilayer tablet according to the present invention generally contains 10 to 160 mg, preferably 20 to 80 mg, of telmisartan and 6.25 to 50 mg, preferably 12.5 to 25 mg, of diuretic. Presently preferred forms are bilayer tablets comprising 40/12.5 mg, 80/12.5 mg and 80/25 mg of telmisartan and HCTZ, respectively.

The first tablet layer contains telmisartan in substantially amorphous form dispersed in a dissolving tablet matrix having immediate release (fast dissolution) characteristics. The dissolving tablet matrix may have acidic, neutral or basic properties, although a basic tablet matrix is preferred.

In such preferred embodiments, the dissolving matrix comprises a basic agent, a water-soluble diluent and, optionally, other excipients and adjuvants.

Specific examples of suitable basic agents are alkali metal hydroxides such as NaOH and KOH; basic amino acids such as arginine and lysine; and meglumine (N-methyl-D-glucamine), NaOH and meglumine being preferred.

Specific examples of suitable water-soluble diluents are carbohydrates such as monosaccharides like glucose; oligosaccharides like sucrose, anhydrous lactose and lactose monohydrate; and sugar alcohols like sorbitol, mannitol, dulcitol, ribitol and xylitol. Sorbitol is a preferred diluent.

The other excipients and/or adjuvants are, for instance, selected from binders, carriers, fillers, lubricants, flow control agents, crystallization retarders, solubilizers, coloring agents, pH control agents, surfactants and emulsifiers, specific examples of which are given below in connection with the second tablet layer composition. The excipients and/or adjuvants for the first tablet layer composition are preferably chosen such that a non-acidic, fast dissolving tablet matrix is obtained.

The first tablet layer composition generally comprises 3 to 50 wt.%, preferably 5 to 35 wt.%, of active ingredient; 0.25 to 20 wt.%, preferably 0.40 to 15 wt.%, of basic agent; and 30 to 95 wt.%, preferably 60 to 80 wt.% of water-soluble diluent.

Other (optional) constituents may, for instance, be chosen from one or more of the following excipients and/or adjuvants in the amounts indicated:
10 to 30 wt.%, preferably 15 to 25 wt.%, of binders, carriers and fillers, thereby replacing the water-soluble diluent;
0.1 to 5 wt.%, preferably 0.5 to 3 wt.%, of lubricants;
0.1 to 5 wt.%, preferably 0.3 to 2 wt.%, of flow control agents;
1 to 10 wt.%, preferably 2 to 8 wt.%, of crystallization retarders;
1 to 10 wt.%, preferably 2 to 8 wt.%, of solubilizers;
0.05 to 1.5 wt.%, preferably 0.1 to 0.8 wt.%, of coloring agents;
0.5 to 10 wt.%, preferably 2 to 8 wt.%, of pH control agents;
0.01 to 5 wt.%, preferably 0.05 to 1 wt.%, of surfactants and emulsifiers.

The second tablet layer composition contains a diuretic in a fast disintegrating tablet matrix. In a preferred embodiment, the disintegrating tablet matrix comprises a filler, a binder, a disintegrant and, optionally, other excipients and adjuvants.

The filler is preferably selected from anhydrous lactose, spray-dried lactose and lactose monohydrate.

The binder is selected from the group of dry binders and/or the group of wet granulation binders, depending on the manufacturing process chosen for the second tablet layer. Suitable dry binders are, e.g., cellulose powder and microcrystalline cellulose. Specific examples of wet granulation binders are corn starch, polyvinyl pyrrolidone (Povidon), vinylpyrrolidone-vinylacetate copolymer (Copovidone) and cellulose derivatives like hydroxymethylcellulose, hydroxyethylcellulose, hydroxypropylcellulose and hydroxypropylmethylcellulose.

Suitable disintegrants are, e.g., sodium starch glycolate, Crospovidon, Croscarmellose, sodium carboxymethylcellulose and dried corn starch, sodium starch glycolate being preferred.

The other excipients and adjuvants, if used, are preferably selected from diluents and carriers such as cellulose powder, microcrystalline cellulose, cellulose derivatives like hydroxymethylcellulose, hydroxyethylcellulose, hydroxypropylcellulose and hydroxy-propylmethylcellulose, dibasic calcium phosphate, corn starch, pregelatinized starch, polyvinyl pyrrolidone (Povidone) etc.; lubricants such as stearic acid, magnesium stearate, sodium stearylfumarate, glycerol tribehenate, etc.; flow control agents such as colloidal silica, talc, etc.; crystallization retarders such as Povidone, etc.; solubilizers such as Pluronic, Povidone, etc.; coloring agents, including dyes and pigments such as Iron Oxide Red or Yellow, titanium dioxide, talc, etc.; pH control agents such as citric acid, tartaric acid, fumaric acid, sodium citrate, dibasic calcium phosphate, dibasic sodium phosphate, etc.; surfactants and emulsifiers such as Pluronic, polyethylene glycols, sodium carboxymethyl cellulose, polyethoxylated and hydrogenated castor oil, etc.; and mixtures of two or more of these excipients and/or adjuvants.

The second tablet layer composition generally comprises 1.5 to 35 wt.%, preferably 2 to 15 wt.%, of active ingredient; 25 to 75 wt.%, preferably 35 to 65 wt.%, of filler; 10 to 40 wt.%, preferably 15 to 35 wt.%, of dry binder; 0.5 to 5 wt.%, preferably 1 to 4 wt.%, of wet granulation binder; and 1 to 10 wt.%, preferably 2 to 8 wt.%, of disintegrant. The other excipients and adjuvants are generally employed in the same amount as in the first tablet layer composition.

For preparing the bilayer tablet according to the present invention, the first and second tablet layer compositions may be compressed in the usual manner in a bilayer tablet press, e.g. a high-speed rotary press in a bilayer tableting mode. However, care should be taken not to employ an excessive compression force for the first tablet layer. Preferably, the ratio of the compression force applied during compression of the first tablet layer to the compression force applied during compression of both the first and second tablet layers is in the range of from 1:10 to 1:2. For instance, the first tablet layer may be compressed at moderate force of 4 to 8 kN, whereas the main compression of first plus second layer is performed at a force of 10 to 20 kN.

During bilayer tablet compression adequate bond formation between the two layers is achieved by virtue of distance attraction forces (intermolecular forces) and mechanical interlocking between the particles.

The bilayer tablets obtained release the active ingredients rapidly and in a largely pH-independent fashion, with complete release occurring within less than 60 min and release of the major fraction occurring within less than 15 min. The dissolution/- disintegration kinetics of the bilayer tablet may be controlled in different ways. For instance, both layers may dissolve/disintegrate simultaneously. Preferably, however, the second tablet layer containing the diuretic disintegrates first whereas the first tablet layer containing telmisartan dissolves in parallel or subsequently.

With the inventive bilayer tablets, a substantially increased dissolution rate of the active ingredients and, in particular, of telmisartan is achieved. Normally, at least 70% and typically at least 90% of the drug load are dissolved after 30 min.

The bilayer tablets of the present invention tend to be slightly hygroscopic and are therefore preferably packaged using a moisture-proof packaging material such as aluminium foil blister packs, or polypropylene tubes and HDPE bottles which preferably contain a desiccant.

For optimum dissolution/disintegration and drug release properties, a specific method of producing the bilayer tablet according to the present invention has been developed which method comprises
(i) providing a first tablet layer composition by
   a) preparing an aqueous solution of telmisartan, at least one basic agent and, optionally, a solubilizer and/or a crystallization retarder;
   b) spray-drying said aqueous solution to obtain a spray-dried granulate;
   c) mixing said spray-dried granulate with a water-soluble diluent to obtain a premix;
   d) mixing said premix with a lubricant to obtain a final blend for the first layer;
   e) optionally, adding other excipients and/or adjuvants in any of steps a) to d);
(ii) providing a second tablet layer composition by
   f) mixing and /or granulating a diuretic with the constituents of a disintegrating tablet matrix and, optionally, further excipients and/or adjuvants;
   g) admixing a lubricant to obtain a final blend for the second tablet layer;
(iii) introducing the first or the second tablet layer composition into a tablet press;
(iv) compressing said tablet layer composition to form a tablet layer;
(v) introducing the other tablet layer composition into the tablet press; and
(vi) compressing both tablet layer compositions to form a bilayer tablet.

An aqueous alkaline solution of telmisartan is prepared by dissolving the active ingredient in purified water with the help of one or more basic agents like sodium hydroxide and meglumine. Optionally, a solubilizer and/or a recrystallization retarder may be added. The dry matter content of the starting aqueous solution is generally 10 to 40 wt.%, preferably 20 to 30 wt.%.

The aqueous solution is then spray-dried at room temperature or preferably at increased temperatures of, for instance, between 50 and 100°C in a co-current or countercurrent spray-drier at a spray pressure of, for instance, 1 to 4 bar. Generally speaking, the spray-drying conditions are preferably chosen in such a manner that a spray-dried granulate having a residual humidity of ≤ 5 wt.%, preferably ≤ 3.5 wt.%, is obtained in the separation cyclone. To that end, the outlet air temperature of the spray-drier is preferably kept at a value of between about 80 and 90°C while the other process parameters such as spray pressure, spraying rate, inlet air temperature, etc. are adjusted accordingly.

The spray-dried granulate obtained is preferably a fine powder having the following particle size distribution:
d₁₀ : ≤ 20 µm, preferably ≤ 10 µm
d₅₀ : ≤ 80 µm, preferably 20 to 55 µm
d₉₀ : ≤ 350 µm, preferably 50 to 150 µm

After spray-drying, the active ingredient (telmisartan) as well as the excipients contained in the spray-dried granulate are in a substantially amorphous state with no crystallinity being detectable. From a physical point of view, the spray-dried granulate is a solidified solution or glass having a glass transition temperature Tg of preferably > 50°C, more preferably > 80°C.

Based on 100 parts by weight of active ingredient (telmisartan), the spray-dried granulate preferably contains 5 to 200 parts by weight of basic agent and, optionally, solubilizer and/or crystallization retarder.

The water-soluble diluent is generally employed in an amount of 30 to 95 wt.%, preferably 60 to 80 wt.%, based on the weight of the first tablet layer composition.

The lubricant is generally added to the premix in an amount of 0.1 to 5 wt.%, preferably 0.3 to 2 wt.%, based on the weight of the first tablet layer composition.

Mixing is carried out in two stages, i.e. in a first mixing step the spray-dried granulate and the diluent are admixed using , e.g., a high-shear mixer or a free-fall blender, and in a second mixing step the lubricant is blended with the premix, preferably also under conditions of high shear. The method of the invention is however not limited to these mixing procedures and, generally, alternative mixing procedures may be employed in steps c), d), and also in the subsequent steps f) and g), such as, e.g., container mixing with intermediate screening.

For direct compression, the second tablet layer composition may be prepared by dry-mixing the constituent components, e.g. by means of a high-intensity mixer or a free-fall blender. Alternatively and preferably, the second tablet layer composition is prepared using a wet granulation technique wherein an aqueous solution of a wet granulation binder is added to a premix and subsequently the wet granulate obtained is dried, e.g. in a fluidized-bed dryer or drying chamber. The dried mixture is screened and then a lubricant is admixed, e.g. using a tumbling mixer or free-fall blender, whereafter the composition is ready for compression.

For production of the bilayer tablet according to the present invention, the first and second tablet layer compositions are compressed in a bilayer tablet press, e.g. a rotary press in the bilayer tableting mode, in the manner described above. In order to avoid any cross-contamination between the first and second tablet layers (which could lead to decomposition of HTCZ), any granulate residues have to be carefully removed during tableting by intense suction of the die table within the tableting chamber.

In order to further illustrate the present invention, the following non-limiting examples are given.

### Reference Example 1

| Constituents | | mg / 1.684 mg SD granulate | volatile constituent | kg / batch |
|---|---|---|---|---|
| (01) | Telmisartan | 1.000 | | 45.000 |
| (02) | Sodium hydroxide | 0.084 | | 3.780 |
| (03) | Povidone K 25 | 0.300 | | 13.500 |
| (04) | Meglumine | 0.300 | | 13.500 |
| (05) | Purified water | | 5.000 | (225.000) |
| | | 1.684 | 5.000 | 75.780 |

### Manufacturing:

### 1. Spray solution

225.000 kg of purified water are measured into a suitable stainless steel vessel at a temperature of between 20-40°C. In sequence, 3.780 of kg sodium hydroxide, 45.000 kg of telmisartan (mixture of polymorph A and B), 13.500 kg of Povidone K 25 and 13.500 kg of meglumine are dissolved in the purified water under intensive stirring until a virtually clear, slightly yellowish, alkaline solution is obtained.

### 2. Spray drying

The solution is sprayed into a suitable spray dryer, e.g. a Niro P 6.3 equipped with Schlick atomizing nozzles of 1.0 mm diameter, with a flow-through heating coil connected upstream of the dryer, and dried to give a white to off-white fine granulate. The spray mode is counter-current at a spray-pressure of about 3 bar, an inlet air temperature of about 125°C and a spray rate of about 11 kg/h, thus resulting in an outlet air temperature of about 85°C. The temperature of the flow through heating coil water bath is set at a temperature of about 80°C.

### 3. Protective Screening

The dry granulate powder is screened through a screen of 0.5 mm mesh size, e.g. using a Vibra Sieve machine.

The resulting amorphous telmisartan spray-dried granulate may be further processed to telmisartan mono-tablets or the first layer of the said bilayer tablet composition.

### Example 2

| | Constituents | mg/tablet 1st layer | mg/SD granulate | mg/tablet 2nd layer |
|---|---|---|---|---|
| (01) | Telmisartan SD granulate | 67.360 | | |
| | consisting of (02) to (06): | | | |
| (02) | Telmisartan | | 40.000 | |
| (03) | Sodium hydroxide | | 3.360 | |
| (04) | Polyvidone (Kollidon 25) | | 12.000 | |
| (05) | Meglumine | | 12.000 | |
| (06) | Purified water | | 264.000* | |
| (07) | Sorbitol P/6 | 168.640 | | |
| (08) | Magnesium stearate, screened | 4.000 | | 1.000 |
| (09) | Hydrochlorothiazide | | | 12.500 |
| (10) | Microcrystalline cellulose (Avicel PH 101) | | | 64.000 |
| (11) | Red iron oxide | | | 0.330 |
| (12) | Sodium starch glycolate | | | 4.000 |
| (13) | Lactose monohydrate fine, screened | | | 112.170 |
| (14) | Maize starch, dried at 45 °C | | | 6.000 |
| | | 240.000 | 67.360 | 200.000 |

| | | | | |
|---|---|---|---|---|
| * 200 mg in SD granulate, 64 mg in granulation liquid of HCTZ granulate | | | | |

### Manufacturing:

### 1. Final blend A

168.640 kg of sorbitol are mixed with 67.360 kg of telmisartan spray dried granulate in a suitable high shear mixer, e.g. Diosna P 600, for 4 minutes using both impeller and chopper. Next 4.0 kg of magnesium stearate are added to the resulting pre-mix and admixed in the high shear mixer for further 30 seconds.

### 2. Final blend B

9.000 kg of purified water of about 70°C are transfered to a suitable mixing vessel, 6.000 kg of maize starch, dried at 45°C, are suspended in the water. This suspension is stirred into 55.000 kg of purified water of about 90°C using e.g. an Ekato stirrer.

Next, 112.170 kg of lactose monohydrate, 12.500 kg of hydrochlorothiazide, 64.000 kg of microcrystalline cellulose (Avicel PH 101), 0.330 kg of red iron oxide and 4.000 kg of sodium starch glycolate are mixed in a suitable high shear granulator, e.g. Diosna P 600, until homogeneous, and moistened with 70.000 kg of the above-prepared aqueous granulating liquid.

Process parameters for wet granulation:

| Process step | Duration (min) | Impeller (setting) | Chopper (setting) |
|---|---|---|---|
| Pre-mixing | 3 | 1 | 1 |
| Moistening | 2 | 1 | 1 |
| Wet mixing | 4 | 2 | 2 |
| Emptying | About 0.5 | 1 | 0 |

After moistening, the resulting wet granulate is dried in a suitable fluid bed dryer, e.g. Glatt WSG 120 at an inlet air temperature of 100°C, an inlet air flow of 2000-3000 m³/h until a product temperature of about 55°C is reached.

The dry granulate is screened to reduce the particle size using a suitable screening machine, e.g. a Comil screen machine equipped with a rasp screen of 2 mm mesh size. Finally 1.000 kg of prescreened magnesium stearate are admixed to the screened granulate material and mixed in a suitable tumbling mixer, e.g. a Lermer rotating spike mixer, for 100 revolutions at a speed of 8-10 rpm.

### 3. Bilaver tablet compression

Using a suitable rotary tablet press, 240 kg of the final blend (A) and 200 kg of the final blend (B) are compressed into bilayer tablets. The target weight for the first layer is 240 mg, the target weight for the second layer is 200 mg.

Process parameters for tableting:

| Tablet press | Fette 3090 | |
|---|---|---|
| Tabletting speed | 100.000 (80.000 - 120.000) tabl./h | |
| Stirrer blade speed: | 1st layer about 30 rpm | 2nd layer about 75 rpm |
| Compression force | 5(4-6)KN | 12(10-14)KN |

As a rule, the tablet hardness is adjusted by variation of the main compression force of the second layer.

The resulting bilayer tablets have the following characteristics:

| | |
|---|---|
| Shape / diameter | oval, both faces convex / 14 x 6.8 mm |
| Colour | first layer: white to off-white second layer: red |
| Weight | 440 mg (total) |
| | 240 mg (layer 1: with telmisartan) |
| | 200 mg (layer 2: with hydrochlorothiazide) |
| Thickness | about 5.2 mm |
| Hardness | about 120 N |
| Disintegration time | NMT 15 min (total) |

### Example 3

| | Constituents | mg/tablet 1st layer | mg/SD granulate | mg/tablet 2nd layer |
|---|---|---|---|---|
| (01) | Telmisartan SD granulate | 67.360 | | |
| | consisting of (02) to (06): | | | |
| (02) | Telmisartan | | 40.000 | |
| (03) | Sodium hydroxide | | 3.360 | |
| (04) | Polyvidone (Kollidon 25) | | 12.000 | |
| (05) | Meglumine | | 12.000 | |
| (06) | Purified water | | (200.000) | |
| (07) | Sorbitol P/6 | 168.640 | | |
| (08) | Magnesium stearate, screened | 4.000 | | 1.000 |
| (09) | Hydrochlorothiazide | | | 25.000 |
| (10) | Microcrystalline cellulose (Avicel PH 101) | | | 64.000 |
| (11) | Yellow iron oxide | | | 0.330 |
| (12) | Sodium starch glycolate | | | 4.000 |
| (13) | Lactose monohydrate fine, screened | | | 105.67 |
| | | 240.000 | 67.360 | 200.000 |

### Manufacturing:

Manufacturing is carried out as in Example 2. Instead of the wet granulation process described in Example 2, the second layer composition is manufactured by dry mixing of (09) to (13) in a suitable free fall blender, e.g. a 1 m³ container mixer, for 200 revolutions at a speed of 10 rpm. Then, (08) is admixed to the main mixture for further 50 revolutions in the container mixer. In order to achieve a homogenous distribution of the color pigment, an additional premix with yellow iron oxide and a portion of the microcrystalline cellulose, e.g. 2.000 kg, which is screened through an 0.8 mm mesh screen manually before transfer to the main mixture, may be performed. The resulting bilayer tablets display virtually the same physical characteristics as described in example 2, except for the color.

### Example 4

Composition of Telmisartan/Hydrochlorothiazide Bilayer Tablets (mg per tablet):

| **Ingredient** | **40/12.5 mg** | **80/12.5 mg** |
|---|---|---|
| **Telmisartan layer** | | |
| Telmisartan | 40.000 | 80.000 |
| Sodium hydroxide | 3.360 | 6.720 |
| Povidone | 12.000 | 24.000 |
| Meglumine | 12.000 | 24.000 |
| Purified water* | (200.000) | (400.000) |
| Sorbitol | 168.640 | 337.280 |
| Magnesium stearate | 4.000 | 8.000 |
| Total telmisartan layer | 240.000 | 480.000 |

| **Hydrochlorothiazide layer** | | |
|---|---|---|
| Hydrochlorothiazide | 12.500 | 12.500 |
| Lactose monohydrate | 112.170 | 112.170 |
| Microcrystalline Cellulose | 64.000 | 64.000 |
| Corn starch | 6.000 | 6.000 |
| Red iron oxide | 0.330 | 0.330 |
| Sodium starch glycolate | 4.000 | 4.000 |
| Purified water* | (64.000) | (64.000) |
| Magnesium stearate | 1.000 | 1.000 |
| Total HCTZ layer | 200.000 | 200.000 |
| **Total tablet weight** | 440.000 | 680.000 |

| | | |
|---|---|---|
| *Does not appear in final produc | | |

t

## Claims

1. A bilayer pharmaceutical tablet for use in a method for the treatment of hypertension comprising
a first layer containing telmisartan in at least 90% amorphous form as determined by X-ray powder diffraction measurement in a dissolving tablet matrix comprising a basic agent and a water-soluble diluent, and
a second layer containing a thiazide diuretic in a disintegrating tablet matrix.

2. A bilayer pharmaceutical tablet as claimed in claim 1 for use in a method for the treatment of hypertension wherein the diuretic is hydrochlorothiazide.

3. A bilayer pharmaceutical tablet as claimed in claim 1 for use in a method for the treatment of hypertension where the basic agent is selected from alkali metal hydroxides, basic amino acids and meglumine.

4. A bilayer pharmaceutical tablet as claimed in claims 1 or 3 for use in a method for the treatment of hypertension wherein the water-soluble diluent is selected from carbohydrates such as monosaccharides like glucose; oligosaccharides like sucrose and lactose; and sugar alcohols like sorbitol, mannitol, dulcitol, ribitol and xylitol.

5. A bilayer pharmaceutical tablet as claimed in claim 1 for use in a method for the treatment of hypertension wherein the dissolving tablet matrix comprises other excipients and adjuvants.

6. A bilayer pharmaceutical tablet as claimed in claim 5 for use in a method for the treatment of hypertension wherein the other excipients and adjuvants are selected from binders, carriers, fillers, lubricants, flow control agents, crystallization retarders, solubilizers, coloring agents, pH control agents, surfactants and emulsifiers.

7. A bilayer pharmaceutical tablet as claimed in any one of claims 1-6 for use in a method for the treatment of hypertension wherein the first tablet layer has been produced by spray-drying an aqueous solution comprising telmisartan and a basic agent to obtain a spray-dried granulate, mixing said spray-dried granulate with a water-soluble diluent to obtain a premix, mixing said premix with a lubricant to obtain a final blend and compressing the final blend to form the first tablet layer.

8. A bilayer pharmaceutical tablet as claimed in any one of claims 1-7 for use in a method for the treatment of hypertension wherein the disintegrating tablet matrix comprises a filler, a binder and a disintegrant.

9. A bilayer pharmaceutical tablet as claimed in claim 1 for use in a method for the treatment of hypertension wherein the disintegrating tablet matrix comprises other excipients and adjuvants.

10. A bilayer pharmaceutical tablet as claimed in claim 9 for use in a method for the treatment of hypertension wherein the other excipients and adjuvants are selected from carriers, diluents, lubricants, flow control agents, solubilizers, coloring agents, pH control agents, surfactants and emulsifiers.

11. A bilayer pharmaceutical tablet as claimed in any one of claims 1-10 for use in a method for the treatment of hypertension containing 10 to 160 mg of telmisartan and 6.25 to 50 mg of thiazide diuretic.

12. A bilayer pharmaceutical tablet as claimed in claim 11 for use in a method for the treatment of hypertension containing 20 to 80 mg of telmisartan and 12.5 to 25 mg of thiazide diuretic.

13. A bilayer pharmaceutical tablet as claimed in any one of claims 1-11 for use in a method for the treatment of hypertension packaged in a moisture proof packaging material such as aluminium foil blister packs, or polypropylene tubes and HDPE bottles.

## Patentansprüche

1. Zweilagige pharmazeutische Tablette zur Verwendung in einem Verfahren zur Behandlung von Hypertonie, umfassend:
eine erste Schicht, enthaltend Teltmisartan in mindestens 90% amorpher Form, wie bestimmt durch röntgenpulverdiffraktometrische Messung, in einer sich auflösenden Tablettenmatrix, umfassend ein basisches Mittel und ein wasserlösliches Verdünnungsmittel, und
eine zweite Schicht, enthaltend ein Thiaziddiuretikum in einer sich zersetzenden Tablettenmatrix.

2. Zweilagige pharmazeutische Tablette nach Anspruch 1 zur Verwendung in einem Verfahren zur Behandlung von Hypertonie, wobei das Diuretikum Hydrochlorthiazid darstellt.

3. Zweilagige pharmazeutische Tablette nach Anspruch 1 zur Verwendung in einem Verfahren zur Behandlung von Hypertonie, wobei das basische Mittel ausgewählt ist aus Alkalirnetallhydroxiden, basischen Aminosäuren und Meglumin.

4. Zweilagige pharmazeutische Tablette nach den Ansprüchen 1 oder 3 zur Verwendung in einem Verfahren zur Behandlung von Hypertonie, wobei das wasserlösliche Verdünnungsmittel ausgewählt ist aus Kohlenhydraten, wie Monosacchariden, wie Glukose; Oligosacchariden, wie Saccharose und Lactose; und Zuckeralkoholen, wie Sorbitol, Mannitol, Dulcitol, Ribitol und Xylitol.

5. Zweilagige pharmazeutische Tablette nach Anspruch 1 zur Verwendung in einem Verfahren zur Behandlung von Hypertonie, wobei die sich auflösende Tablettenmatrix andere Zusatzstoffe und Hilfsmittel umfasst.

6. Zweilagige pharmazeutische Tablette nach Anspruch 5 zur Verwendung in einem Verfahren zur Behandlung von Hypertonie, wobei die anderen Zusatzmittel und Hilfsstoffe ausgewählt sind aus Bindemitteln, Trägem, Füllstoffe, Schmiermitteln, Flusskontrollmitteln, Kristallisationsverzögerern, Lösungshilfsmitteln, farbgebenden Mitteln, pH-Kontrollmitteln, oberflächenaktiven Mitteln und Emulgatoren.

7. Zweilagige pharmazeutische Tablette nach irgendeinem der Ansprüche 1 bis 6 zur Verwendung in einem Verfahren zur Behandlung von Hypertonie, wobei die erste Tablettenschicht hergestellt wird durch Sprühtrocknen einer wässerigen Lösung, umfassend Telmisartan und ein basisches Mittel, um ein sprühgetrocknetes Granulat zu erhalten, Mischen des sprühgetrockneten Granulats mit einem wasserlöslichen Verdünnungsmittel, um eine Vormischung zu erhalten, Mischen der Vormischung mit einem Schmiermittel, um eine Endmischung zu erhalten, und Komprimieren der Endmischung, um die erste Tablettenschicht zu bilden.

8. Zweilagige pharmazeutische Tablette nach irgendeinem der Ansprüche 1 bis 7 zur Verwendung in einem Verfahren zur Behandlung von Hypertonie, wobei die sich zersetzende Tablettenmatrix einen Füllstoff, ein Bindemittel und ein Zerfallshilfsmittel enthält.

9. Zweilagige pharmazeutische Tablette nach Anspruch 1 zur Verwendung in einem Vierfahren zur Behandlung von Hypertonie, wobei die sich zersetzende Tablettenmatrix andere Zusatzstoffe und Hilfsmittel umfasst.

10. Zweilagige pharmazeutische Tablette nach Anspruch 9 zur Verwendung in einem Verfahren zur Behandlung von Hypertonie, wobei die anderen Zusatzstoffe und Hilfsmittel ausgewählt sind aus Trägern, Verdünnungsmitteln, Schmiermitteln, Flusskontrollmitteln, Lösungshilfsmitteln, farbgebenden Mitteln, pH-Kontrollmitteln, oberflächenaktiven Mitteln und Emulgatoren.

11. Zweilagige pharmazeutische Tablette nach irgendeinem der Ansprüche 1 bis 10 zur Verwendung in einem Verfahren zur Behandlung von Hypertonie, enthaltend 10 bis 160 mg Telmisartan und 6,25 bis 50 mg Thiaziddiuretikum.

12. Zweilagige pharmazeutische Tablette nach Anspruch 11 zur Verwendung in einem Vierfahren zur Behandlung von Hypertonie, enthaltend 20 bis 80 mg Telmisartan und 12,5 bis 25 mg Thiaziddiuretikum.

13. Zweilagige pharmazeutische Tablette nach irgendeinem der Ansprüche 1 bis 11 zur Verwendung in einem Verfahren zur Behandlung von Hypertonie, verpackt in einem feuchtigkeitsdichten Verpackungsmaterial, wie Aluminiumfolienblisterpackungen oder Polypropylenröhrchen und HDPE-Flaschen.

## Revendications

1. Comprimé pharmaceutique bicouche destiné à être utilisé dans un procédé pour le traitement de l'hypertension comprenant
une première couche contenant du telmisartan sous une forme amorphe à au moins 90 % comme déterminé par mesure de diffraction des rayons X de poudre dans une matrice de comprimé dissolvante comprenant un agent basique et un diluant soluble dans l'eau, et
une seconde couche contenant un diurétique thiazide dans une matrice de comprimé désintégrante.

2. Comprimé pharmaceutique bicouche selon la revendication 1 destiné à être utilisé dans un procédé pour le traitement de l'hypertension où le diurétique est l'hydrochlorothiazide.

3. Comprimé pharmaceutique bicouche selon la revendication 1 destiné à être utilisé dans un procédé pour le traitement de l'hypertension où l'agent basique est choisi parmi les hydroxydes de métaux alcalins, les aminoacides basiques et la méglumine.

4. Comprimé pharmaceutique bicouche selon la revendication 1 ou 3 destiné à être utilisé dans un procédé pour le traitement de l'hypertension où le diluant soluble dans l'eau est choisi parmi les glucides comme les monosaccharides tels que le glucose ; les oligosaccharides tels que le saccharose et le lactose ; et les sucres-alcools tels que le sorbitol, le mannitol, le dulcitol, le ribitol et le xylitol.

5. Comprimé pharmaceutique bicouche selon la revendication 1 destiné à être utilisé dans un procédé pour le traitement de l'hypertension où la matrice de comprimé dissolvante comprend d'autres excipients et adjuvants.

6. Comprimé pharmaceutique bicouche selon la revendication 5 destiné à être utilisé dans un procédé pour le traitement de l'hypertension où les autres excipients et adjuvants sont choisis parmi les liants, les vecteurs, les charges, les lubrifiants, les agents de régulation de l'écoulement, les retardateurs de cristallisation, les solubilisants, les agents colorants, les agents de régulation du pH, les tensioactifs et les émulsifiants.

7. Comprimé pharmaceutique bicouche selon l'une quelconque des revendications 1-6 destiné à être utilisé dans un procédé pour le traitement de l'hypertension où la première couche du comprimé a été produite par séchage par pulvérisation d'une solution aqueuse comprenant du telmisartan et un agent basique pour obtenir des granulés séchés par pulvérisation, le mélange desdits granulés séchés par pulvérisation avec un diluant soluble dans l'eau pour obtenir un prémélange, le mélange dudit prémélange avec un lubrifiant pour obtenir un mélange final et la compression du mélange final pour former la première couche du comprimé.

8. Comprimé pharmaceutique bicouche selon l'une quelconque des revendications 1-7 destiné à être utilisé dans un procédé pour le traitement de l'hypertension où la matrice de comprimé désintégrante comprend une charge, un liant et un désintégrant.

9. Comprimé pharmaceutique bicouche selon la revendication 1 destiné à être utilisé dans un procédé pour le traitement de l'hypertension où la matrice de comprimé désintégrante comprend d'autres excipients et adjuvants.

10. Comprimé pharmaceutique bicouche selon la revendication 9 destiné à être utilisé dans un procédé pour le traitement de l'hypertension où les autres excipients et adjuvants sont choisis parmi les vecteurs, les diluants, les lubrifiants, les agents de régulation de l'écoulement, les solubilisants, les agents colorants, les agents de régulation du pH, les tensioactifs et les émulsifiants.

11. Comprimé pharmaceutique bicouche selon l'une quelconque des revendications 1-10 destiné à être utilisé dans un procédé pour le traitement de l'hypertension contenant 10 à 160 mg de telmisartan et 6,25 à 50 mg de diurétique thiazide.

12. Comprimé pharmaceutique bicouche selon la revendication 11 destiné à être utilisé dans un procédé pour le traitement de l'hypertension contenant 20 à 80 mg de telmisartan et 12,5 à 25 mg de diurétique thiazide.

13. Comprimé pharmaceutique bicouche selon l'une quelconque des revendications 1-11 destiné à être utilisé dans un procédé pour le traitement de l'hypertension conditionné dans un matériau de conditionnement à l'épreuve de l'humidité comme les blisters à feuille d'aluminium ou les tubes en polypropylène et les flacons en HDPE.
